(19) Europäisches Patentamt
European Patent Office
Office européen des brevets

(11) **EP 4 027 352 A1**

(12) **EUROPEAN PATENT APPLICATION**
published in accordance with Art. 153(4) EPC

(43) Date of publication:
**13.07.2022 Bulletin 2022/28**

(21) Application number: **20860494.2**

(22) Date of filing: **03.09.2020**

(51) International Patent Classification (IPC):
*G16H 40/20* (2018.01)   *G16H 80/00* (2018.01)
*G06Q 50/22* (2018.01)   *G16H 50/50* (2018.01)
*G16H 50/70* (2018.01)   *G06Q 10/06* (2012.01)

(52) Cooperative Patent Classification (CPC):
**G06Q 10/06; G06Q 50/22; G16H 40/20;
G16H 50/50; G16H 50/70; G16H 80/00**

(86) International application number:
**PCT/KR2020/011872**

(87) International publication number:
**WO 2021/045535 (11.03.2021 Gazette 2021/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.09.2019 KR 20190109299
04.09.2019 KR 20190109300**

(71) Applicant: **Ontact Health Co., Ltd.
Seoul 03764 (KR)**

(72) Inventors:
• **CHANG, Hyuk Jae
Seoul 03722 (KR)**
• **PARK, Eun Jeong
Seoul 03722 (KR)**

• **SUNG, Ji Min
Seoul 07700 (KR)**
• **KIM, Ji Hoon
Seoul 04129 (KR)**
• **KIM, Min Joung
Seoul 08807 (KR)**
• **KIM, Sung Woo
Incheon 22709 (KR)**
• **JEON, Byung Hwan
Seoul 03654 (KR)**
• **HA, Seong Min
Seoul 05343 (KR)**
• **ANN, Kyeong Jin
Seoul 03721 (KR)**

(74) Representative: **Fabry, Bernd
IP2 Patentanwalts GmbH
Schlossstrasse 523-525
41238 Mönchengladbach (DE)**

(54) **OPTIMAL TRANSFER HOSPITAL DETERMINING METHOD AND SERVER**

(57)    An optimal transfer hospital determining method and server are provided. Provided is the optimal transfer hospital determining method comprising: determining candidate hospitals; acquiring status information about an emergency patient; determining the severity of the patient on the basis of the acquired status information; calculating emergency event possibility information on the basis of the acquired status information; acquiring transport resources availability information about the determined candidate hospitals; calculating the suitability of each candidate hospital on the basis of the determined severity of the patient, the acquired emergency event possibility information, and the transport resource availability information; and determining the optimal transfer hospital on the basis of the calculated suitability of each candidate hospital.

| DETERMINE CANDIDATE HOSPITALS (S210) |
| --- |
| ACQUIRE STATUS INFORMATION ABOUT EMERGENCY PATIENT (S220) |
| DETERMINE SEVERITY OF PATIENT(S230) |
| CALCULATE EMERGENCY EVENT POSSIBILITY INFORMATION (S240) |
| ACQUIRE TRANSPORT RESOURCES AVAILABILITY INFORMATION (S250) |
| CALCULATE SUITABILITY OF EACH CANDIDATE HOSPITAL (S260) |
| DETERMINE FINAL TRANSFER HOSPITAL (S270) |

**FIG. 2**

EP 4 027 352 A1

**Description**

[Technical Field]

The present invention provides a method for determining an optimal transfer hospital of an emergency patient when an emergency patient occurs and a server providing the same.

[Background Art]

Currently, according to the domestic emergency medical system, it is difficult to provide high-quality emergency medical services because information flow between hospitals, emergency sites, and control organizations, which are participants in the emergency medical system, is cut off, and there is a limit to collecting and using integrated data at emergency sites. This causes inefficiency in an emergency treatment, the transport, and the hospital transfer of severely ill patients which require appropriate in-site judgement and causes a serious problem in an emergency medical service which must ensure public health and safety.

Specifically, when a patient transfer hospital is selected, situation information which changes in real-time between the 119 main control center and the emergency site is not smoothly transmitted (due to information exchange through voice communication) and the time delay is caused. Further, when the transfer hospital is selected, several calls to individual emergency medical centers are necessary so that the information transmitted through the voice communication causes the difficulty to transmit accurate information about the patient's condition.

This causes the time delay of the emergency treatment for the severely ill patient and after the transfer of the severe emergency disease, causes a primary hospital transfer rate of 11.2% and a secondary hospital transfer rate of 8.6%.

Further, due to the difficulty in categorizing the severity of the prehospital stage, 30.7% of severe cardiovascular diseases, 31.9% of severe brain-nervous system diseases, and 44.6% of severe trauma were transferred to inappropriate medical institutions and appropriate treatment was not provided.

Further, non-professionals intervene in the decision of the transfer hospital and transfer to a hospital that can provide appropriate treatment is frequently failed.

In the domestic medical situation, it is difficult to select an optimal transfer hospital due to overcrowding of emergency rooms, errors in categorizing severity, and problems with road conditions so that it is necessary to develop a technology for solving these problems.

Registered Patent No. KR10-0800026 discloses a method and a system for transferring an emergency patient to an optimal medical institution.

[Detailed Description of the Invention]

[Technical Problems]

The present invention has been contrived to cope with the above-described background art and an object is to efficiently and accurately determine an optimal transfer hospital for an emergency patient when an emergency patient occurs.

[Means for Solving the Problems]

In order to achieve the above-described object, a first aspect of the exemplary embodiments of the present invention may provide an optimal transfer hospital determining method including: determining candidate hospitals; acquiring status information about an emergency patient; determining a severity of the patient on the basis of the acquired status information; calculating emergency event possibility information on the basis of the acquired status information; acquiring transport resources availability information about the determined candidate hospitals; calculating the suitability of each candidate hospital on the basis of the determined severity of the patient, the acquired emergency event possibility information, and the transport resource availability information; and determining an optimal transfer hospital on the basis of the calculated suitability of each candidate hospital.

Further, the status information of the emergency patient includes at least one of biosignal information, age information, complained symptom information, existing medical history information, consciousness information, and electrocardiogram information, the emergency event possibility information includes at least one of intensive care unit hospitalization possibility information, STEMI possibility information, UA + NSTEM possibility information, LVO possibility information, cerebral infarction and cerebral hemorrhage possibility information, return of spontaneous circulation possibility information, and cardiac arrest recurrence possibility information, and the transport resource availability information may include at least one of real-time traffic information, location information of each candidate hospital, current position information, available sickbed information of each candidate hospital, duty doctor information of each candidate hospital, facility information of each candidate hospital, air ambulance location information, and air ambulance operation information.

Further, when the determined suitability of the optimal transfer hospital is lower than a predetermined value, the method further includes re-determining candidate hospitals by expanding a search radius; calculating a suitability of the re-determined candidate hospitals; and re-determining an optimal transfer hospital on the basis of the calculated suitability of each candidate hospital.

Further, when the re-determined suitability of the optimal transfer hospital is lower than a predetermined value, the following steps (1), (2), and (3) may be continuously repeated until the re-determined suitability of the optimal transfer hospital becomes equal to or higher than a predetermined value: (1) re-determining candidate hospitals by expanding a search radius; (2) calculating a suitability of the re-determined candidate hospitals; and (3) re-determining an optimal transfer hospital on the basis of the calculated suitability of each candidate hospital.

Further, the method may further include: determining whether to utilize an air ambulance to transport the emergency patient on the basis of at least one of the determined location information of the optimal transfer hospital, real-time traffic information, and air ambulance operation information, and determining an optimal handover point on the basis of at least one of position information of the determined transfer hospital, real-time traffic information, and air ambulance operation information when the air ambulance is utilized to transport the emergency patient.

In order to achieve the above-described object, a first aspect of the exemplary embodiments of the present invention may provide an optimal transfer hospital determining server including a control unit which determines a severity of a patient on the basis of acquired status information about an emergency patient, calculates an emergency event possibility information, calculates a suitability of each candidate hospital on the basis of the determined severity of the patient, the emergency event possibility information, and transport resource availability information, and determines an optimal transfer hospital on the basis of the calculated suitability of each candidate hospital; and a storage unit which stores the status information of the emergency patient, the emergency event possibility information, and the transport resource availability information.

Further, the status information of the emergency patient includes at least one of biosignal information, age information, complained symptom information, existing medical history information, consciousness information, and electrocardiogram information, the emergency event possibility information includes at least one of intensive care unit hospitalization possibility information, STEMI possibility information, UA + NSTEM possibility information, LVO possibility information, cerebral infarction and cerebral hemorrhage possibility information, return of spontaneous circulation possibility information, and cardiac arrest recurrence possibility information, and the transport resource availability information may include at least one of real-time traffic information, location information of each candidate hospital, current position information, available sickbed information of each candidate hospital, duty doctor information of each candidate hospital, facility information of each candidate hospital, air ambulance location information, and air ambulance operation information.

Further, when the determined suitability of the optimal transfer hospital is lower than a predetermined value, the control unit re-determines candidate hospitals by expanding a search radius, calculates a suitability of re-determined candidate hospitals, and may re-determine an optimal transfer hospital on the basis of the calculated suitability of each candidate hospital.

Further, when the re-determined suitability of the optimal transfer hospital is lower than a predetermined value, the following steps (1), (2), and (3) may be continuously repeated until the re-determined suitability of the optimal transfer hospital becomes equal to or higher than the predetermined value: (1) re-determining candidate hospitals by expanding a search radius; (2) calculating a suitability of the re-determined candidate hospitals; and (3) re-determining an optimal transfer hospital on the basis of the calculated suitability of each candidate hospital.

In order to achieve the above-described object, a second aspect of the exemplary embodiments of the present invention may provide a method for determining a hospital to which each of a plurality of emergency patients is transported, including: determining candidate hospitals; generating weight information about each candidate hospital of each emergency patient; determining an optimal transfer hospital of each emergency patient; and inquiring the determined optimal transfer hospital about whether to accept the emergency patient, together with weight information.

Further, in the generating weight information, a weight for each candidate hospital of each emergency patient may be calculated by transport distance based hospital modeling and patient information based modeling.

Further, when the determined optimal transfer hospital does not accept the emergency patient as a result of inquiring about whether to accept the emergency patient, the weight information may be regenerated after updating information about the emergency patients and the candidate hospitals in real-time.

Further, the determining a final transfer hospital of each emergency patient includes: acquiring status information about an emergency patient; determining a severity of the patient on the basis of the acquired status information; calculating emergency event possibility information on the basis of the acquired status information; acquiring transport resources availability information about the determined candidate hospitals; calculating the suitability of each candidate hospital on the basis of the determined severity of the patient, the acquired emergency event possibility information, and the transport resource availability information; and determining an optimal transfer hospital on the basis of the calculated suitability of each candidate hospital.

Further, the status information of the emergency patient includes at least one of biosignal information, age information, complained symptom information, existing medical history information, consciousness information, and electrocardiogram information, the emergency event possibility information includes at least one of intensive care unit hospitalization possibility information, STEMI possibility information, UA + NSTEM possibility information, LVO possibility information, cerebral infarction and cerebral hemorrhage possibility information, return of spontaneous circulation possibility information, and cardiac arrest recurrence possibility information, and the transport resource availability information may include at least one of real-time traffic information, location information of each candidate hospital, current position information, available sickbed information of each candidate hospital, duty doctor information of each candidate hospital, facility information of each candidate hospital, air ambulance location information, and air ambulance operation information.

Further, when the determined suitability of the optimal transfer hospital is lower than a predetermined value, the method may further include: re-determining candidate hospitals by expanding a search radius; calculating a suitability of the re-determined candidate hospitals; and re-determining an optimal transfer hospital on the basis of the calculated suitability of each candidate hospital.

Further, when the re-determined suitability of the optimal transfer hospital is lower than a predetermined value, the following steps (1), (2), and (3) may be continuously repeated until the re-determined suitability of the optimal transfer hospital becomes equal to or higher than the predetermined value: (1) re-determining candidate hospitals by expanding a search radius; (2) calculating a suitability of the re-determined candidate hospitals; and (3) re-determining an optimal transfer hospital on the basis of the calculated suitability of each candidate hospital.

Further, the method further includes: determining whether to utilize an air ambulance to transport the emergency patient on the basis of at least one of the determined location information of the optimal transfer hospital, real-time traffic information, and air ambulance operation information, and determining an optimal handover point on the basis of at least one of the location information of the determined optimal transfer hospital, real-time traffic information, and air ambulance operation information when the air ambulance is utilized to transport the emergency patient.

In order to achieve the above-described object, a second aspect of the exemplary embodiments of the present invention provides a server for determining a hospital to which each of a plurality of emergency patients is transported including a control unit which determines candidate hospitals, generates weight information for candidate hospitals of each emergency patient, determines an optimal transfer hospital of each emergency patient, and inquires the determined optimal transfer hospital about whether to accept the emergency patient together with the weight information.

Further, the control unit determines a severity of a patient on the basis of the acquired status information of the emergency patient, calculates emergency event possibility information, calculates a suitability of each candidate hospital on the basis of the determined severity of the patient, emergency event possibility information, and the transport resource availability information, and determines an optimal transfer hospital on the basis of the calculated suitability of each candidate hospital, and the server for determining a hospital to which each of a plurality of emergency patients is transported further includes a storage unit which stores the status information of the emergency patient, the emergency event possibility information, and the transport resource availability information, the status information of the emergency patient includes at least one of biosignal information, age information, complained symptom information, existing medical history information, consciousness information, and electrocardiogram information, the emergency event possibility information includes at least one of intensive care unit hospitalization possibility information, STEMI possibility information, UA + NSTEM possibility information, LVO possibility information, cerebral infarction and cerebral hemorrhage possibility information, return of spontaneous circulation possibility information, and cardiac arrest recurrence possibility information, and the transport resource availability information includes at least one of real-time traffic information, location information of each candidate hospital, current position information, available sickbed information of each candidate hospital, duty doctor information of each candidate hospital, facility information of each candidate hospital, air ambulance location information, and air ambulance operation information.

[Effects of the Invention]

The present invention has been contrived to cope with the above-described background art and provides a method for determining an optimal transfer hospital for an emergency patient.

[Brief Description of the Drawings]

Various aspects will be described with reference to the drawings and like reference numerals collectively designate like elements. In the following exemplary embodiments, a plurality of specific details will be suggested for more understanding of one or more aspects for the purpose of description. However, it will be apparent that the aspect(s) will be embodied without having the specific details. In other examples, known structures and devices will be illustrated as a block diagram to easily describe the one or more aspects.

FIG. 1 illustrates an optimal transfer hospital determining system according to an exemplary embodiment of the present disclosure.

FIG. 2 illustrates a method for determining an optimal transfer hospital according to an exemplary embodiment of the present disclosure.

FIG. 3 is a view for explaining a method for re-determining an optimal transfer hospital according to an exemplary embodiment of the present disclosure.

FIG. 4 is a view for explaining a method for determining an optimal transfer hospital according to an exemplary embodiment of the present disclosure when a plurality of emergency patients occurs.

FIG. 5 is a view for explaining a method for recommending an optimal handover point according to an exemplary embodiment of the present disclosure.

FIG. 6 is a view for explaining an algorithm for determining an optimal transfer hospital according to an exemplary embodiment of the present disclosure in detail.

FIG. 7 is a view for explaining a component of an emergency AI server according to an exemplary embodiment of the present disclosure.

[Best Modes for Carryng out the Invention]

Various exemplary embodiments will be described with reference to the drawings and like reference numerals denote like components throughout the drawings. In the specification, various descriptions are suggested to provide understanding of the present invention. However, it is obvious that the exemplary embodiments may be embodied without having the specific description. In other examples, known structures and devices are provided as a block diagram to easily describe the exemplary embodiments.

Terms such as "component', "module", or "system" used in the specification indicate a computer-related entity, hardware, firmware, software, a combination of software and hardware, or execution of software. For example, a component may be a processing step which is executed in a processor, a processor, an object, an execution thread, a program and/or a computer, but is not limited thereto. For example, both an application which is executed in a computing apparatus and a computing apparatus may be a component. One or more components may be stayed in a processor and/or an executing thread, one component may be localized in one computer or distributed between two or more computers. Further, such components may be executed from various computer readable media having various data structures stored therein. The components may communicate with each other through local and/or remote processings in accordance with, for example, a signal including one or more data packets (for example, data through other system and a network, such as an Internet, through data and/or a signal from one component which interacts with other component in a local system or a distributed system).

In the present specification, an ambulance device 1000 may include an arbitrary type of computer system or computer device such as a microprocessor, a main frame computer, a digital single processor, a portable device, and a device controller which are used in the ambulance.

In the present specification, an emergency AI server 3000 may refer to a single server. The emergency AI server 3000 may also refer to a group configured by a plurality of servers. Further, the emergency AI server 3000 may refer to a cloud server, but is not limited thereto.

In the present specification, an emergency medical server 2000 may refer to a single server. The emergency medical server 2000 may also refer to a group configured by a plurality of servers. Further, the emergency medical server 2000 may refer to a cloud server, but is not limited thereto.

Description of the suggested exemplary embodiments is provided to allow those skilled in the art to use or embody the present invention. Various modifications of the exemplary embodiments may be apparent to those skilled in the art and general principles defined herein may be applied to other exemplary embodiments without departing from the scope of the present invention. Therefore, the present invention is not limited to the exemplary embodiments suggested herein, but should be interpreted in the broadest range which is consistent with principles suggested herein and new features.

Hereinafter, exemplary embodiments of the present invention will be described in detail with reference to the accompanying drawings.

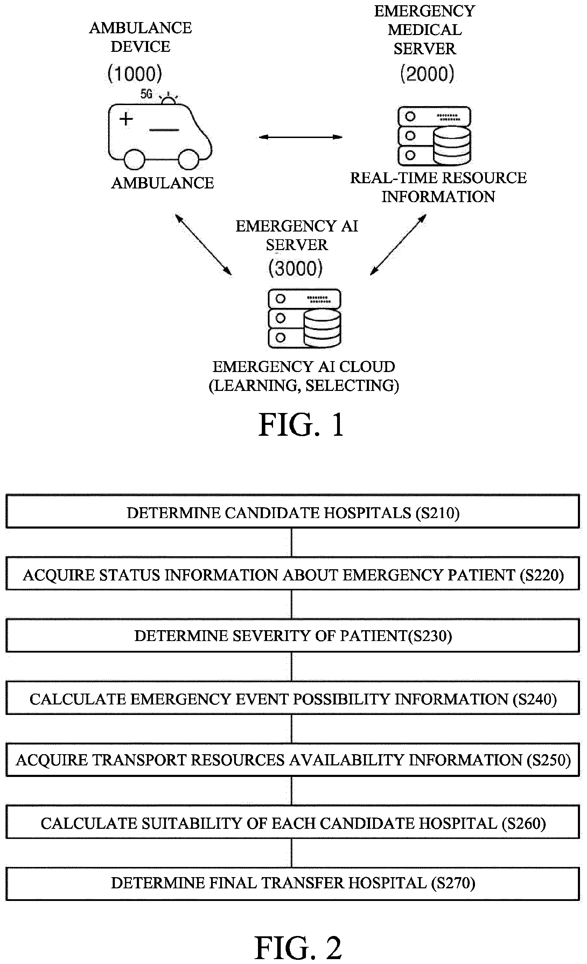

FIG. 1 illustrates an optimal transfer hospital determining system according to an exemplary embodiment of the present disclosure.

According to an exemplary embodiment of the present disclosure, when an emergency patient occurs, paramedics (for example, 119 paramedics) may be dispatched to the corresponding area. When the emergency patient is in the ambulance, paramedics may give first aid and obtain information about the emergency patient.

For example, the paramedics may create an emergency activity log, record the voice of the emergency patient, and use a camera to take a picture of the emergency patient's condition. Further, the paramedic may measure biosignals of the emergency patient using various devices equipped in the ambulance.

Various status information about the emergency patient acquired by the paramedics may be stored in the ambulance device 1000. In this case, the ambulance device 1000 may include various devices which store information of the emergency patient and communicate with external devices, such as terminals used by the paramedics or computers equipped in the ambulance.

According to the exemplary embodiment of the present disclosure, the ambulance device 1000 may automatically acquire status information of the emergency patient. For example, the ambulance device 1000 may automatically create the emergency activity log by recognizing syllables of the words of the paramedics. Further, the ambulance device 1000 automatically acquires the status information of the emergency patient by analyzing the photographed images.

The ambulance device 1000 transmits the status information of the patient to an emergency artificial intelligence (AI) server 3000 and may request severity of the emergency patient and/or emergency event possibility information. Further, the ambulance device 1000 transmits the status information of the patient and may request the emergency AI server 3000 to determine an optimal transfer hospital.

The emergency AI server 3000 inputs the status information of the patient to a previously generated severity determination model in response to the request of the ambulance device 1000 to determine the severity of the patient and may provide the determined severity to the ambulance device 1000. Further, the emergency AI server 3000 inputs the status information of the patient to a previously generated machine learning model in response to the request of the ambulance device 1000 to generate severe illness event possibility information and may provide the generated information to the ambulance device 1000.

Further, the emergency AI server 3000 determines the optimal transfer hospital for the emergency patient in response to the request of the ambulance device 1000 and may provide information about the determined optimal transfer hospital to the ambulance device 1000.

The emergency medical server 2000 may include various servers which provide information related to the emergency patient. For example, the emergency medical server 2000 may include National Emergency Department Information Service (NEDIS) server. The emergency medical server 2000 may acquire and include various information related to the emergency medical service and provide the information to the ambulance device 1000 and/or the emergency AI server 3000.

For example, the emergency medical server 2000 may acquire and contain at least one of real-time traffic information, location information of each hospital, current position information, available sickbed information of each hospital, duty doctor information of each hospital, facility information of each candidate hospital, air ambulance location information, and air ambulance operation information, and may also acquire and include various information without being limited thereto. Further, the emergency medical server may provide the acquired information to the ambulance device 1000 and/or the emergency AI server 3000.

FIG. 2 illustrates a method for determining an optimal transfer hospital according to an exemplary embodiment of the present disclosure.

In step S210, the emergency AI server 3000 may determine candidate hospitals for the optimal transfer hospital. For example, the emergency AI server 3000 may determine hospitals located within a predetermined distance (for example, 3 Km, 5 Km, or the like) from a location where the ambulance is located (or a location where an emergency patient occurs) as the candidate hospitals. Further, the emergency AI server 3000 may determine hospitals located in the city (for example, Seoul, Incheon, Suwon, or the like) including a location where the ambulance is located as the candidate hospitals. Further, the emergency AI server 3000 may determine hospitals located in the district (for example, Gangnam-gu, Seocho-gu, Gangdong-gu, or the like) where the ambulance is located as the candidate hospitals, but may determine the candidate hospitals by various methods without being limited thereto.

In step S220, the emergency AI server 3000 may acquire status information about the emergency patient. For example, the emergency AI server 3000 may acquire the status information about the emergency patient from the ambulance device 1000.

The status information of the emergency patient may include at least one of biosignal information, age information, complained symptom information, existing medical history information, consciousness information, and electrocardiogram information, but is not limited thereto.

According to the exemplary embodiment of the present disclosure, in step S230, the emergency AI server 3000 may determine the severity of the emergency patient. For example, the emergency AI server 3000 may determine the severity of the patient using the status information of the emergency patient.

In this case, the emergency AI server 3000 may previously create (or previously include) a severity determination model through machine learning and input the status information of the emergency patient to the severity determination model to determine the severity of the emergency patient.

In this case, the emergency AI server 3000 may determine the degree of emergency of the emergency patient on the basis of a prehospital Korean Triage and Acuity Scale (prehospital KTAS). For example, the emergency AI server 3000 primarily assigns a code by utilizing an age of the emergency patient, secondarily assigns a code by performing a major classification for symptom (for example, a digestive system, dermatology, neurology, or the like), tertiarily assigns a code by performing a middle classification for the major classification (for example, abdominal pain, vomiting/nausea, or the like), and quaternarily assigns a code by determining detailed symptoms (for example, dyspnea, shock, or the like) for the middle classification. Further, the emergency AI server 3000 may determine a severity (for example, levels 1, 2, 3, 4, and 5 and the level 1 is the most dangerous) matching the finally assigned code as the severity of the emergency patient.

In this case, the algorithm utilized to create the severity determination model may include at least one of supervised learning, support vector machines (SVM), random forest (RF), naive bayes (NB), artificial neural networks (ANN), decision tree, and Bayesian, but is not limited thereto.

In step S240, the emergency medical server 2000 may calculate emergency event possibility information using the status information of the emergency patient.

In this case, the emergency event possibility information may include at least one of intensive care unit hospitalization possibility information, ST-elevation myocardial infarction (STEMI) possibility information, unstable angina (UA) and non ST-elevation myocardial infarction (NSTEMI) possibility information, large vessel occlusion (LVO) possibility information, cerebral infarction and cerebral hemorrhage possibility information, return of spontaneous circulation (ROSC) possibility information, and cardiac arrest recurrence possibility information.

According to the exemplary embodiment of the present disclosure, the emergency AI server 3000 may acquire the event possibility information by inputting the status information of the emergency patient to the machine learning model generated by the machine learning in advance. For example, the emergency AI server 3000 may generate an intensive care unit hospitalization screening model in advance and inputs the status information of the emergency patient to the intensive care unit hospitalization screening model to acquire the intensive care unit hospitalization possibility information. Further, the emergency AI server 3000 may generate a STEMI screening model in advance and inputs the status information of the emergency patient to the STEMI screening model to acquire the STEMI possibility information. Further, the emergency AI server 3000 may generate at least one of a UA and NSTEMI screening model, a LVO occurrence screening model, a cerebral infarction and cerebral hemorrhage screening model, a return of spontaneous circulation (ROSC) screening model, and a cardiac arrest recurrence screening model and input the status information of the emergency patient to each model to acquire at least one of unstable angina (UA) and non ST-elevation myocardial infarction (NSTEMI) possibility information, large vessel occlusion (LVO) possibility information, cerebral infarction and cerebral hemorrhage possibility information, return of spontaneous circulation (ROSC) possibility information, and cardiac arrest recurrence possibility information.

In this case, each screening model may be implemented by a Bayesian deep hierarchical network model which models simultaneously the inherent uncertainty of data and interprets the results, and also implemented by various methods without being limited thereto. For example, the screening model may be implemented by one of supervised learning, support vector machines (SVM), random forest (RF), naive bayes (NB), artificial neural networks (ANN), decision tree, Bayesian, and the like, but is not limited thereto.

In this case, the emergency AI server 3000 continuously performs the machine learning by utilizing the data of the emergency patient which is continuously input to improve the accuracy of each screening model.

In step S250, the emergency AI server 3000 may acquire transport resource availability information.

For example, the emergency AI server 3000 may acquire the transport resource availability information from the emergency medical server 2000. Further, the emergency AI server 3000 may acquire the transport resource availability information from the ambulance device 1000. Further, the emergency AI server 3000 may acquire the transport resource availability information from various external devices, without being limited thereto.

The transport resource availability information is information about a resource consumed to transport the emergency patient and may include at least one of real-time traffic information, location information of each candidate hospital, current position information, available sickbed information of each candidate hospital, duty doctor information of each candidate hospital, facility information of each candidate hospital, air ambulance location information, and air ambulance operation information.

In step S260, the emergency AI server 3000 may calculate a suitability of each candidate hospital.

According to the exemplary embodiment of the present disclosure, the emergency AI server 3000 may calculate the suitability of each candidate hospital on the basis of at least one of the determined severity of the patient, the acquired emergency event possibility information, and the transport resource availability information.

For example, the emergency AI server 3000 may generate and include an optimal hospital selection model in advance and input the determined severity of the patient, the emergency event possibility information, and the transport resource availability information to the optimal hospital selection model to calculate the suitability for each candidate hospital.

In this case, the algorithm utilized to create the optimal hospital selection model may include at least one of supervised learning, support vector machines (SVM), random forest (RF), naive bayes (NB), artificial neural networks (ANN), decision tree, and Bayesian, but is not limited thereto.

In step S270, the emergency AI server 3000 may select the optimal transfer hospital.

According to the exemplary embodiment of the present disclosure, the emergency AI server 3000 determines the optimal transfer hospital according to the higher order of the suitability of the candidate hospitals and may provide the information about the determined optimal transfer hospital to the ambulance device 1000. For example, the emergency AI server 3000 determines one hospital having the highest suitability among the candidate hospitals as an optimal transfer hospital and may provide information thereof to the ambulance device 1000. Further, the emergency AI server 3000 determines a predetermined number of hospitals (for example, two, three, or the like) according to the higher order of the suitability among the candidate hospitals as an optimal transfer hospital and may provide information thereof to the ambulance device 1000.

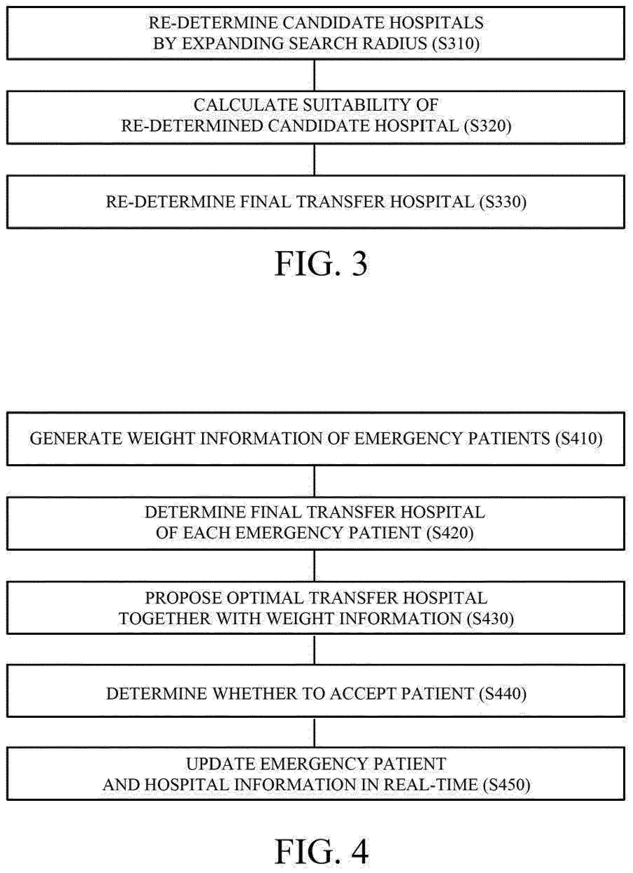

FIG. 3 is a view for explaining a method for re-determining an optimal transfer hospital according to an exemplary embodiment of the present disclosure.

According to the exemplary embodiment of the present disclosure, when the determined suitability of the optimal transfer hospital is lower than a predetermined value, the emergency AI server 3000 may re-determine the optimal transfer hospital.

In step S310, the emergency AI server 3000 may re-determine candidate hospitals by expanding a search radius. For example, the emergency AI server 3000 may re-determine candidate hospitals by expanding a search radius (for example, increasing by 2 km, expanding the cities, expanding the region, or the like) from the location of the ambulance (or a location where the emergency patient occurs).

In step S320, the emergency AI server 3000 may calculate suitabilityes of re-determined candidate hospitals. For example, the emergency AI server 3000 may reacquire the transport resource availability information by reflecting the re-determined candidate hospitals and recalculate the suitability of each re-determined candidate hospital. The method for calculating the suitability has been described in FIG. 2 so that a detailed description thereof will be omitted.

In step S330, the emergency AI server 3000 may re-determine the optimal transfer hospital.

According to the exemplary embodiment of the present disclosure, the emergency AI server 3000 determines the optimal transfer hospital according to the higher order of the suitability among the re-determined candidate hospitals and may provide the information about the re-determined optimal transfer hospital to the ambulance device 1000. For example, the emergency AI server 3000 re-determines one hospital having the highest suitability among the candidate hospitals as an optimal transfer hospital and may provide information thereof to the ambulance device 1000.

According to the exemplary embodiment of the present disclosure, when the suitability of the re-determined optimal transfer hospital is lower than a predetermined value, the step S310 of re-determining candidate hospitals by expanding the search radius, the step S320 of calculating a suitability of the re-determined candidate hospitals, and the step S330 of re-determining the optimal transfer hospital on the basis of the calculated suitability of each candidate hospital may be continuously repeated until the suitability of the re-determined optimal transfer hospital becomes equal to or higher than the predetermined value.

FIG. 4 is a view for explaining a method for determining an optimal transfer hospital according to an exemplary embodiment of the present disclosure when a plurality of emergency patients occurs.

When emergency patients having similar symptoms occur within a predetermined distance at similar timings, if only an individual optimal hospital selection module is utilized, all patients are likely to be transported to the same hospital.

According to the exemplary embodiment of the present disclosure, referring to s410, when a plurality of emergency patients occurs within a predetermined time, in a predetermined distance, the emergency AI server 3000 may generate weight information about each candidate hospital for each emergency patient using information about the emergency patients and information about the candidate hospital.

A weight for the candidate hospitals for each emergency patient may be explained by the following Equation.

$$w_t^{(i,j,k_0)} \leftarrow w_{t-1}^{(i,j,k_0)}\, p\left(z_t^{(i,j,k_0)}\middle|x_t^{(i,j,k_0)}\right)$$

This equation may be explained in detail by $p\left(x_{0:t}^{(i,j,k)}\middle|z_{1:t}\right) = p\left(z_t\middle|x_t^{(i,j,k)}\right) p\left(x_{0:t-1}^{(i,j,k)}\middle|z_{1:t-1}\right)$ and the part $p\left(z_t\middle|x_t^{(i,j,k)}\right)$ may be embodied by the following Equation.

$$p\left(x_{0:t}^{(i,j,k)}\middle|z_{1:t}\right) = p\left(z_t\middle|x_t^{(i,j,k)}\right) \underbrace{p\left(x_{0:t-1}^{(i,j,k)}\middle|z_{1:t-1}\right)}$$

$$p(z_t|x_t) \propto p\left(z_t\middle|\delta_t^{(j,k)}, \phi_t^{(j,k)}, \kappa_t^{(i)}, \rho_t^{(i)}\right)$$

$$= p\left(z_{\delta,t}\middle|\delta_t^{(j,k)}\right) p\left(z_{\phi,t}\middle|\phi_t^{(j,k)}, \kappa_t^{(i)}, \rho_t^{(i)}\right)$$

As a result, the weight of each emergency patient may be calculated by the following Equation.

$$\underbrace{p\left(z_{\delta,t}\middle|\delta_t^{(j,k)}\right)}_{\substack{\text{Transport distance} \\ \text{based hospital modeling}}} \underbrace{p\left(z_{\phi,t}\middle|\phi_t^{(j,k)}, \kappa_t^{(i)}, \rho_t^{(i)}\right)}_{\substack{\text{Patient information} \\ \text{based hospital modeling}}}$$

When it is explained again, the emergency AI server 3000 may calculate the weight for the candidate hospital of each emergency patient by 1) transport distance based hospital modeling and 2) patient information based hospital modeling.

In this case, variables will be explained as follows.

| Symbols | Meanings |
|---|---|
| $i$ | Patient index |
| $j$ | Hospital index |
| $k$ | Lesion index |
| $g^k$ | Golden time period (or distance) for lesion k |
| $c_1$ | Approval state (1/0) |
| $\delta$ | Transport distance (proportional to taken time) |
| $c_2$ | Sickbed situation (1/0) |
| $c_3$ | Request to refrain from transporting (1/0) |
| $\phi^{k=1}$ | Cardiac arrest hospitalization survival rate |
| $\phi^{k=2}/\phi^{max}$ | Major trauma: number of complete recovery/maximum number of complete recovery |
| $\phi^{k=3}$ | STEMI acceptance rate |
| $\phi^{k=4}$ | Stroke acceptance rate |
| $\kappa$ | K-TAS grade |
| $\rho$ | Whether to be hospitalized in intensive care unit [0, 1] |
| $c_4$ | Event occurrence 1 |
| $c_5$ | Event occurrence 2 |
| $c_6$ | Event occurrence 3 |

1) Transport distance based hospital modeling

The transport distance is closely related to golden time information corresponding to the representative severe disease. Accordingly, the weight for the candidate hospital of the emergency patient may be calculated on the basis of the transport distance information which is calculated together with hospital (j) and lesion (k) factor information.

2) Patient information based hospital modeling

The weight for the candidate hospital of the emergency patient may be calculated on the basis of the patient information $\varphi$ about at least one of the cardiac arrest hospitalization survival rate, the major trauma, the STEMI acceptance rate, and the stroke acceptance rate, the lesion $\kappa$, and whether to be hospitalized in the intensive care unit $\rho$.

When the method for calculating a weight for the candidate hospital of each emergency patient is explained according to the exemplary embodiment of the present disclosure in detail, the weight may be calculated by the following Equations.

1) Example of transport distance based modeling

$$p\left(z_{\delta,t}\middle|\delta_t^{(j,k)}\right) = \begin{cases} 0 & , \text{if } \delta_t^{(j)} > g^{(k)} + \epsilon \\ \alpha_1\left\{2\left(\frac{\delta_t^{(j)}}{g^{(k)}-\Delta t}\right)^3 - 3\left(\frac{\delta_t^{(j)}}{g^{(k)}-\Delta t}\right)^2 + 1\right\} & \text{otherwise} \end{cases}$$

where $\Delta t = t - t_{1st\ call}$

2) Example of patient information based modeling

$$p\left(z_{\phi,t}\middle|\phi_t^{(j,k)},\kappa_t^{(i)},\rho_t^{(i)}\right) = \underbrace{c_1^{(j,k)}c_2^{(j,k)}(1-c_3^{(j,k)})c_4^{(i)}c_5^{(i)}c_6^{(i)}}_{\text{Event condition}}\underbrace{\frac{1}{\alpha_2\sqrt{2\pi}}}_{\substack{\text{Optimization}\\\text{parameter}}}e^{-0.5\left[\left[1-\rho_t^{(i)}\phi_t^{(j,k)}\left(1-\frac{\kappa_t^{(i)}}{4}\right)\right]/\alpha_2\right]^2}$$

Proficiency, Severity, kTAS information — Optimization parameter

First, if a probability is zero in 1),

When a probability is zero in 1), it means that the survival probability is slim so that finally, as the weight of the real-time patient which is updated by 1), 2), and the joint operation

$$\left(w_t^{(i,j,k_0)} \leftarrow w_{t-1}^{(i,j,k_0)} p\left(z_t^{(i,j,k_0)}\middle|x_t^{(i,j,k_0)}\right)\right)$$

of the previous weight, a total weight $w_t^{(i,j,k_0)}$ is zero because the probability in 1) is zero. When the patients flock to the same hospital (assumed situation), a patient list to which the weight is reflected is transmitted to a specific hospital A and patient information of a patient whose weight becomes 0 may disappear from the real-time weight list. Accordingly, when the patient information disappears, the function of assigning the patient to a hospital is lost, which causes exception to the assumed situation.

As a result, a patient whose weight becomes 0 is excluded from a list in which weights of all real-time patients are sorted to be excluded from the hospital list.

Second, in 2), if it is affected by a conditional variable c

In 2), like the first assumption, a real-time patient weight may become 0 by the conditions $c1, c2, c3, c4, c5$, and $c6$. However, this case is simply related to the acceptance of the hospital such as the approval of the hospital, a sickbed situation, refraining from transporting so that a process of quickly searching a hospital as a second best option is necessary.

When the weight of the patient becomes 0 by the conditional variable before re-searching a hospital, like the first assumption, the patient information disappears from the hospital list. Accordingly, exception to the assumption situation is caused.

Third, if the weight is not 0

In 1) and 2), the weight may not be 0. In this case, the patient information in the hospital list is not removed so that an available range is updated in real-time by the hospital to sequentially process. Here, the process of updating the available range in real-time is partially associated with the second assumption. (When the hospital may not accept, $c1$, $c2, c3 = 0$ so that the patient information possessed by the hospital disappears)

That is, in the assumed situation, patients which are found by the emergency crew are put in the list of the hospital A according to the order of First-in-first-out and the algorithm proceeds in such a way that the hospital A accepts the acceptable patient, but excess patients are allocated to a next hospital.

In step S420, the emergency AI server 3000 may determine a final transfer hospital for each emergency patient. The method for determining an optimal transfer hospital has been described above so that a detailed description will be omitted here.

In step S430, the emergency AI server 3000 may inquire the determined optimal transfer hospital about whether to accept the patient together with the weight information of the emergency patient. Further, the emergency AI server may receive a response to the inquiry from the hospital.

Further, the emergency AI server may provide the information about the determined optimal transfer hospital and the weight information of the emergency patient to the ambulance device 1000 and the ambulance device 1000 may inquire the hospital about whether to accept the patient together with the weight information of the emergency patient.

In step s440, the hospital may determine whether to accept the emergency patient. Further, the hospital may transmit the answer about whether to accept the corresponding patient to the ambulance device and/or the emergency AI server 3000.

The hospital may receive weight information about the plurality of emergency patients and determine an emergency patient to be accepted in consideration of the weight information. Further, the hospital may quickly assign patients who are not accepted to another hospital in consideration of the weight information.

In step S450, the emergency AI server 3000 may update information about the emergency patients and hospitals in real-time. For example, when a patient having a high weight is assigned to a specific hospital, acceptable patients of the specific hospital may be reduced. In this case, weight information of a patient having a low weight may be regenerated by reflecting the changed information.

According to another exemplary embodiment of the present disclosure, after the determining a final transfer hospital, generating weight information may be performed. Specifically, the emergency AI server 3000 determines a final transfer hospital for each emergency patient first and calculates weight information for the determined final transfer hospital to generate weight information for the final transfer hospital.

According to another exemplary embodiment of the present disclosure, the emergency AI server 3000 may determine the optimal transfer hospital before generating the weight information. For example, the emergency AI server 3000 determines a final transfer hospital for each emergency patient and generates weight information for the determined final transfer hospital, and then may inquire the optimal transfer hospital about whether to accept the patient together with the weight information. In this case, the number of hospitals which generate weight information is reduced so that the efficiency of the emergency AI server 3000 may be improved.

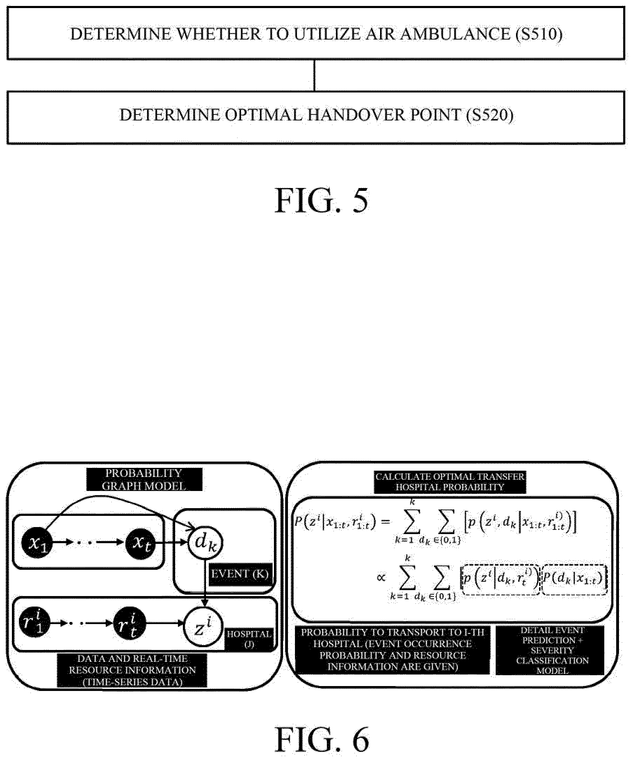

FIG. 5 is a view for explaining a method for recommending an optimal handover point according to an exemplary embodiment of the present disclosure.

According to the exemplary embodiment of the present disclosure, the emergency AI server 3000 may acquire location information of an optimal transfer hospital, real-time traffic information, air ambulance operation information, and information about a candidate handover point (a location where an ambulance hands over an emergency patient to an air ambulance).

In step S510, the emergency AI server 3000 may determine whether to use an air ambulance to transport an emergency patient.

For example, when the emergency patient's condition is urgent so that emergency measures are required or when it takes a lot of time to transport the patient (reflecting regional characteristics), the emergency AI server 3000 may determine to utilize an air ambulance.

When it is determined to utilize the air ambulance, in step S520, the emergency AI server 3000 may determine an optimal handover point on the basis of at least one of location information of the determined optimal transfer hospital, real-time traffic information, location information of a candidate handover point, ambulance location information (or location information where the emergency patient occurs), and air ambulance operation information.

For example, the emergency AI server 3000 may determines candidate handover points and determine a plurality of candidate routes which utilize an air ambulance. For example, the emergency AI server 3000 may determine a candidate route which utilizes the candidate handover points.

Further, the emergency AI server 3000 may calculate a time taken to go to the optimal transfer hospital through each of the plurality of candidate routes. For example, the emergency AI server 3000 may determine a taken time for each of the candidate routes by utilizing location information of the determined optimal transfer hospital, real-time traffic information, location information of a candidate handover point, ambulance location information (or location information where the emergency patient occurs), and air ambulance operation information.

Further, the emergency AI server 3000 may determine a candidate route having a shortest taken time as an optimal transport route.

When the emergency AI server 3000 determines an optimal transport route, the emergency AI server may provide the information to the ambulance device 1000. In this case, the information provided to the ambulance device 1000 may include information about the optimal handover point.

FIG. 6 is a view for explaining an algorithm for determining an optimal transfer hospital according to an exemplary embodiment of the present disclosure in detail.

Referring to FIG. 6, $P\left(z^i = 1 \middle| x_{1:t}, r^i_t\right)$ may indicate a suitability of an i-th hospital when data during transport and current in-hospital resource information are given. This may be calculated by a conditional probability to be disclosed below. $P(d_k|x_{1:t})$ may indicate emergency event possibility information and a severity classification result when data during transport is given. $P\left(z^i \middle| d_k, r^i_t\right)$ indicates a suitability of an i-th hospital when emergency event possibility information and transport resource availability information are given.

The following table represents examples of input variables and output variables.

1. Example of input variable and output variable for $P(d_k|x_{1:t})$

[Table 1]

| Classification | List | Type | Remarks |
|---|---|---|---|
| Input variable (x^t) | Biosignal, age | Signal (7-dimensional vector) | |
| | Main complained symptom, existing medical history information | Natural language | |
| | consciousness information | {A, V, P, U} | |
| | 12 lead electrocardiogram | Signal (12-dimensional time-series data) | |
| Output variable (d_k) | 7 event prediction/screeni ng probability (entering ICU, STEMI, or the like) | {0, 1} binary value for 7 events | Calculated by AI model |
| | Severity (KTAS) | {1, 2, 3, 4, 5} non-continuity value | |

2. Example of input variable and output variable for $P\left(z^i \middle| d_k, r^i_t\right)$

[Table 2]

| Classification | List | Type | Remarks |
|---|---|---|---|
| Input variable ($d_k$) | KTAS | {1, 2, 3, 4, 5} non-continuity value | Identify whether current input variable occurs through $P(d_k\|x_{1:t})$ modeling |
| | 7 event occur (entering ICU, STEMI, or the like) | {0, 1} binary value for 7 events | |
| Input variable ($r^i_t$) | Reporting time | MM-DD HH:MM:SS | |
| | Patient location | Latitude-longitude | |
| | Resource availability information | {0,1} binary value | |
| Output variable ($z^i$) | Transport hospital | {0,1} binary value for n hospitals | Design conditional probability by utilizing golden time information for every disease and real-time resource information |

3. Example of input variable and output variable for $P\left(z^i = 1 \middle| x_{1:t}, r^i_t\right)$

[Table 3]

| Classification | List | Type | Data source |
|---|---|---|---|
| Input variable (Xt) | Biosignal, age | Signal (seven-dimensional vector) | Emergency activity log |
| | Main complained symptom, existing medical history information | Natural language | |
| | consciousness information | {A, V, P, U} | |
| | 12 lead electrocardiogram | Signal (12-dimensional time-series data) | |
| Input variable ($r^i_t$) | Reporting time | MM-DD HH:MM:SS | Data linked platform |
| | Patient location | Latitude-longitude | |
| | Resource availability information | {0,1} binary value | |
| Output variable ($z^i$) | Transfer hospital | {0, 1} binary value for n hospitals | Estimate distribution by optimal transfer hospital module |

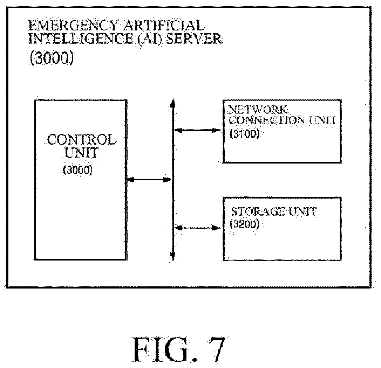

FIG. 7 is a view for explaining components of an emergency AI server according to an exemplary embodiment of the present disclosure.

According to the exemplary embodiment of the present disclosure, the emergency AI server 3000 may include a control unit 3300, a network connection unit 3100, and a storage unit 3200, and may also include various components without being limited thereto.

The storage unit 3200 may include various information. For example, the storage unit 3200 may include received status information of the emergency patient (for example, biosignal information, age information, complained symptom information, existing medical history information, consciousness information, electrocardiogram information, and the like). For example, the storage unit 3200 may include transport resource information (for example, real-time traffic information, location information of each candidate hospital, current position information, available sickbed information of each candidate hospital, duty doctor information of each candidate hospital, facility information of each candidate hospital, air ambulance location information, air ambulance operation information, and the like).

Further, the information stored in the storage unit 3200 may be updated in real-time or non-real-time on the basis of information received from an external device.

The storage unit 3200 may be implemented by a non-volatile storage medium which consistently stores arbitrary data. For example, the storage unit 3200 may include not only a disk, an optical disk, and a magneto-optical storage device, but also a flash memory and/or a battery back-up memory based storage device, but is not limited thereto.

Further, the storage unit 3200 may include various machine learning models. For example, the storage unit 3200 may include at least one of a severity determination model, an intensive care unit hospitalization screening model, a STEMI screening model, a UA and NSTEMI screening model, an LVO occurrence screening model, a cerebral hemorrhage screening model, a ROSC recovery screening model, and a cardiac arrest recurrence screening model. The control unit 3300 can continuously update the machine learning models stored in the storage unit 3200 by performing the machine learning utilizing data acquired from the outside.

The network connection unit 3100 may communicate with the ambulance device 1000 and the emergency medical server 2000 via an arbitrary type of network. The network connection unit 3100 may include a wired/wireless connection module for network connection. As the wireless connection technique, for example, wireless LAN (WLAN) (Wi-Fi), wireless broadband (Wibro), world interoperability for microwave access (Wimax), high speed downlink packet access (HSDPA), international mobile telecommunication system (IMT) 2000, IMT advanced, IMT-2020, and the like may be used.

The control unit 3300 may be implemented by at least one processor. For example, the control unit 3300 may be implemented by one processor or a plurality of processors. Further, when the control unit 3300 is implemented by a plurality of processors, the plurality of processors may be located to be physically adjacent and to be physically spaced apart from each other.

According to the exemplary embodiment of the present disclosure, the control unit 3300 may determine candidate hospitals of the optimal transfer hospital. For example, the control unit 3300 may determine hospitals located within a predetermined distance (for example, 3 Km, 5 Km, or the like) from a location where the ambulance is located (or a location where an emergency patient occurs) as the candidate hospitals. Further, the control unit 3300 may determine hospitals located in the city (for example, Seoul, Incheon, Suwon, or the like) including a location where the ambulance is located as the candidate hospitals. Further, the control unit 3300 may determine hospitals located in the district (for example, Gangnam-gu, Seocho-gu, Gangdong-gu, or the like) where the ambulance is located as the candidate hospitals, but may determine the candidate hospitals by various methods without being limited thereto.

Further, the control unit 3300 may acquire status information about the emergency patient. For example, the control unit 3300 may acquire the status information about the emergency patient from the ambulance device 1000.

The control unit 3300 may determine a severity of the emergency patient. For example, the control unit 3300 may determine the severity of the patient using the status information about the emergency patient.

In this case, the control unit 3300 may previously create (or previously include) a severity determination model through machine learning and determine the severity of the emergency patient by inputting the status information of the emergency patient to the severity determination model.

In this case, the control unit 3300 may determine the severity of the emergency patient on the basis of a prehospital Korean Triage and Acuity Scale (prehospital KTAS). For example, the control unit 3300 primarily assigns a code by utilizing an age of the emergency patient, secondarily assigns a code by performing a major classification for symptom (for example, a digestive system, dermatology, neurology, or the like), tertiarily assigns a code by performing a middle classification for the major classification (for example, abdominal pain, vomiting/nausea, or the like), and quaternarily assigns a code by determining detailed symptoms for the middle classification (for example, dyspnea, shock, or the like). Further, the control unit 3300 may determine a severity matching the finally assigned code as the severity of the emergency patient (for example, levels 1, 2, 3, 4, and 5 and the level 1 is the most dangerous).

In this case, the algorithm utilized to create the severity determination model may include at least one of supervised learning, support vector machines (SVM), random forest (RF), naive bayes (NB), artificial neural networks (ANN), decision tree, and Bayesian, but is not limited thereto.

The emergency AI server 2000 may calculate emergency event possibility information using the status information of the emergency patient.

According to the exemplary embodiment of the present disclosure, the control unit 3300 may acquire the possibility information of the event by inputting the status information of the emergency patient to the machine learning model generated by the machine learning in advance. For example, the control unit 3300 generates (and/or includes) an intensive care unit hospitalization screening model in advance and inputs the status information of the emergency patient to the intensive care unit hospitalization screening model to acquire the possibility information of entering to the intensive care unit. Further, the control unit 3300 generates (and/or includes) a STEMI screening model in advance and inputs the status information of the emergency patient to the STEMI screening model to acquire the STEMI possibility information. Further, the control unit 3300 may generate (and/or include) at least one of a UA and NSTEMI screening model, a LVO occurrence screening model, a cerebral infarction and cerebral hemorrhage screening model, a return of spontaneous circulation (ROSC) screening model, and a cardiac arrest recurrence screening model and input the status information of the emergency patient to each model to acquire at least one of unstable angina (UA) and non ST-elevation myocardial infarction (NSTEMI) possibility information, large vessel occlusion (LVO) possibility information, cerebral infarction and cerebral hemorrhage possibility information, return of spontaneous circulation (ROSC) possibility information, and cardiac arrest recurrence possibility information.

In this case, each screening model may be implemented by a Bayesian deep hierarchical network model which models simultaneously the inherent uncertainty of data and interprets the results, and also implemented by various methods without being limited thereto. For example, the screening model may be implemented by one of supervised learning, support vector machines (SVM), random forest (RF), naive bayes (NB), artificial neural networks (ANN), decision tree, Bayesian, and the like, but is not limited thereto.

In this case, the control unit 3300 may improve the accuracy of each screening model, by performing the machine learning continuously utilizing the data of the emergency patient which is continuously input.

The control unit 3300 may calculate a suitability of each candidate hospital. According to the exemplary embodiment of the present disclosure, the control unit 3300 may calculate the suitability of each candidate hospital on the basis of at least one of the determined severity of the patient, the acquired emergency event possibility information, and the transport resource availability information.

For example, the control unit 3300 may generate and include an optimal hospital selection model in advance and input the determined severity of the patient, the emergency event possibility information, and the transport resource availability information to the optimal hospital selection model to calculate the suitability for each candidate hospital.

The control unit 3300 may select an optimal transfer hospital. According to the exemplary embodiment of the present disclosure, the control unit 3300 determines the optimal transfer hospital according to the higher order of the suitability among the candidate hospitals and may provide the information about the determined optimal transfer hospital to the ambulance device 1000. For example, the control unit 3300 determines one hospital having the highest suitability among the candidate hospitals as an optimal transfer hospital and may provide information thereof to the ambulance device 1000. Further, the control unit 3300 determines a predetermined number of hospitals (for example, two, three, or the like) according to the higher order of the suitability among the candidate hospitals as an optimal transfer hospital and may provide information thereof to the ambulance device 1000.

According to the exemplary embodiment of the present disclosure, when the determined suitability of the optimal transfer hospital is lower than a predetermined value, the control unit 3300 may re-determine the optimal transfer hospital.

The control unit 3300 may re-determine the candidate hospitals by expanding a search radius. For example, the control unit 3300 may re-determine candidate hospitals by expanding a search radius (for example, increasing 2 km, expanding the cities, expanding the region, or the like) from the location of the ambulance (or a location where the emergency patient occurs).

The control unit 3300 may calculate a suitability of re-determined candidate hospitals. For example, the control unit 3300 may reacquire the transport resource availability information by reflecting the re-determined candidate hospitals and may recalculate the suitability of each re-determined candidate hospital. The method for calculating the suitability has been described in FIG. 2 so that a detailed description thereof will be omitted.

The control unit 3300 may re-determine an optimal transfer hospital. According to the exemplary embodiment of the present disclosure, the control unit 3300 determines the optimal transfer hospital according to the higher order of the suitability among the re-determined candidate hospitals and may provide the information about the re-determined optimal transfer hospital to the ambulance device 1000. For example, the control unit 3300 re-determines one hospital having the highest suitability among the candidate hospitals as an optimal transfer hospital and may provide information thereof to the ambulance device 1000.

According to the exemplary embodiment of the present disclosure, when the suitability of the re-determined optimal transfer hospital is lower than a predetermined value, the step S310 of re-determining candidate hospitals by expanding the search radius, the step S320 of calculating a suitability of the re-determined candidate hospitals, and the step S330 of re-determining the optimal transfer hospital on the basis of the calculated suitability of each candidate hospital may be continuously repeated until the suitability of the re-determined optimal transfer hospital becomes equal to or higher than the predetermined value.

When a plurality of emergency patients occurs within a predetermined time, in a predetermined distance, the control unit 3000 may generate weight information about each candidate hospital for each emergency patient using information about the emergency patients and information about the candidate hospitals. This has been described in detail in FIG. 4.

Further, the control unit 3300 may determine a final transfer hospital for each emergency patient. The method for determining an optimal transfer hospital has been described above so that a detailed description will be omitted here.

The control unit 3300 may inquire the determined optimal transfer hospital about whether to accept the patient together with the weight information of the emergency patient. Further, the control unit may receive a response to the inquiry from the hospital.

The hospital may determine whether to accept the emergency patient. Further, the hospital may transmit the answer about whether to accept the patient to the ambulance device and/or the emergency AI server 3300.

The control unit 3300 may update information about emergency patients and hospitals in real-time. For example, when a patient having a high weight is assigned to a specific hospital, acceptable patients of the specific hospital may be reduced. In this case, weight information of a patient having a low weight may be regenerated by reflecting the changed information.

According to the exemplary embodiment of the present disclosure, the control unit 3300 may acquire location information of an optimal transfer hospital, real-time traffic information, operation information of air ambulance, and information about a candidate handover point (a location where an ambulance hands over an emergency patient to an air ambulance).

The control unit 3300 may determine whether to utilize an air ambulance to transport the emergency patient. For example, when the emergency patient's condition is urgent so that emergency measures are required or when it takes a lot of time to transport the patient (reflecting regional characteristics), the control unit 3300 may determine to utilize an air ambulance.

When it is determined to utilize the air ambulance, the control unit 3300 may determine an optimal handover point on the basis of at least one of location information of the determined optimal transfer hospital, real-time traffic information, location information of a candidate handover point, ambulance location information (or location information where the emergency patient occurs), and air ambulance operation information.

For example, the control unit 3300 may determine candidate handover points and determine a plurality of candidate routes which utilize an air ambulance. For example, the control unit 3300 may determine a candidate route which utilizes the candidate handover points.

Further, the control unit 3300 may calculate a time taken to go to the optimal transfer hospital through each of the plurality of candidate routes. For example, the control unit 3300 may determine a taken time for each of the candidate routes by utilizing location information of the determined optimal transfer hospital, real-time traffic information, location information of a candidate handover point, ambulance location information (or location information where the emergency patient occurs), and operation information of air ambulance.

Further, the control unit 3300 may determine a candidate route having a shortest taken time as an optimal transport route.

Those skilled in the art may understand that information and signals may be represented using various arbitrary technologies and techniques. For example, data, indications, commands, information, signals, bits, symbols, and chips which may be referred in the above description may be represented by voltages, currents, electromagnetic waves, magnetic fields or particles, optical fields or particles, or an arbitrary combination thereof.

Those skilled in the art may understand that various exemplary logical blocks, modules, processors, units, circuits, and algorithm steps which have been described with respect to the exemplary embodiments disclosed herein may be implemented by electronic hardware, various types of programs (for the convenience, referred to as "software" here), a design code, or a combination thoseof. In order to clearly describe compatibility of hardware and software, various exemplary components, blocks, modules, circuits, and steps are generally described above with respect to functions thoseof. Whether these functions are implemented as hardware or software is determined depending on design restrictions which are applied to a specific application and the entire system. Those skilled in the art may implement the function, which is described by various methods, of the specific application but the implementation determination is not interpreted to depart from the scope of the present disclosure.

Various exemplary embodiments suggested herein may be implemented by a method, a device, or a standard programing and/or an article using an engineering technique. The term "article" includes a computer program which is accessible from an arbitrary computer readable storage device, a carrier or a medium. For example, the computer readable storage medium includes a magnetic storing device (for example, a hard disk, a floppy disk, a magnetic strip, or the like), an optical disk (for example, a CD, a DVD, or the like), a smart card, and a flash memory device (for example, an EEPROM, a card, a stick, a key drive, or the like), but is not limited thereto. Further, various storage media suggested herein include one or more devices for storing information and/or other machine readable media.

It should be understood that a specific order or a hierarchical structure of steps in suggested processes are examples of exemplary approaches. It should be understood that a specific order or a hierarchical structure of steps in the processes may be rearranged within the scope of the present disclosure, based on a design priority. The accompanying method claims provide elements of various steps in the order of sample, but the claims are not meant to be limited to the suggested specific order or hierarchical structure.

Description of the suggested exemplary embodiment is provided to allow those skilled in the art to use or embody the present disclosure. Various modifications of the exemplary embodiments may be apparent to those skilled in the art and general principles defined herein may be applied to other exemplary embodiments without departing from the scope of the present disclosure. Therefore, the present disclosure is not limited to the exemplary embodiments suggested herein, but interpreted in the broadest range which is consistent with principles suggested herein and new features.

**Claims**

1. An optimal transfer hospital determining method, comprising:

   determining candidate hospitals;
   acquiring status information about an emergency patient;
   determining a severity of the patient on the basis of the acquired status information;
   calculating emergency event possibility information on the basis of the acquired status information;
   acquiring transport resources availability information about the determined candidate hospitals;
   calculating the suitability of each candidate hospital on the basis of the determined severity of the patient, the acquired emergency event possibility information, and the transport resource availability information; and
   determining the optimal transfer hospital on the basis of the calculated suitability of each candidate hospital.

2. The optimal transfer hospital determining method of claim 1, wherein the status information of the emergency patient includes at least one of biosignal information, age information, complained symptom information, existing medical history information, consciousness information, and electrocardiogram information,

   the emergency event possibility information includes at least one of intensive care unit hospitalization possibility information, STEMI possibility information, UA + NSTEM possibility information, LVO possibility information, cerebral infarction and cerebral hemorrhage possibility information, return of spontaneous circulation possibility information, and cardiac arrest recurrence possibility information, and
   the transport resource availability information includes at least one of real-time traffic information, location information of each candidate hospital, current position information, available sickbed information of each candidate hospital, duty doctor information of each candidate hospital, facility information of each candidate hospital, air ambulance location information, and air ambulance operation information.

3. The optimal transfer hospital determining method of claim 2, further comprising:

   when the determined suitability of the optimal transfer hospital is lower than a predetermined value,
   re-determining candidate hospitals by expanding a search radius;
   calculating a suitability of the re-determined candidate hospitals; and
   re-determining an optimal transfer hospital on the basis of the calculated suitability of each candidate hospital.

4. The optimal transfer hospital determining method of claim 3, wherein when the re-determined suitability of the optimal transfer hospital is lower than a predetermined value, the following steps (1), (2), and (3) are continuously repeated until the re-determined suitability of the optimal transfer hospital becomes equal to or higher than the predetermined value:

   (1) re-determining candidate hospitals by expanding a search radius;
   (2) calculating a suitability of the re-determined candidate hospitals; and
   (3) re-determining an optimal transfer hospital on the basis of the calculated suitability of each candidate hospital.

5. The optimal transfer hospital determining method of claim 1, further comprising:

   determining whether to utilize an air ambulance to transport the emergency patient on the basis of at least one of location information of the determined optimal transfer hospital, real-time traffic information, and air ambulance operation information, and
   determining an optimal handover point on the basis of at least one of the location information of the determined optimal transfer hospital, real-time traffic information, and air ambulance operation information when the air ambulance is utilized to transport the emergency patient.

6. An optimal transfer hospital determining server, comprising:

   a control unit which determines a severity of a patient on the basis of acquired status information about an emergency patient, calculates an emergency event possibility information, calculates a suitability of each candidate hospital on the basis of the determined severity of the patient, the emergency event possibility information, and transport resource availability information, and determines an optimal transfer hospital on the basis of the calculated suitability of each candidate hospital; and

a storage unit which stores the status information about the emergency patient, the emergency event possibility information, and the transport resource availability information.

7. The optimal transfer hospital determining server of claim 6, wherein the status information of the emergency patient includes at least one of biosignal information, age information, complained symptom information, existing medical history information, consciousness information, and electrocardiogram information,

the emergency event possibility information includes at least one of intensive care unit hospitalization possibility information, STEMI possibility information, UA + NSTEM possibility information, LVO possibility information, cerebral infarction and cerebral hemorrhage possibility information, return of spontaneous circulation possibility information, and cardiac arrest recurrence possibility information, and
the transport resource availability information includes at least one of real-time traffic information, location information of each candidate hospital, current position information, available sickbed information of each candidate hospital, duty doctor information of each candidate hospital, facility information of each candidate hospital, air ambulance location information, and air ambulance operation information.

8. The optimal transfer hospital determining server of claim 7, wherein when the determined suitability of the optimal transfer hospital is lower than a predetermined value, the control unit re-determines candidate hospitals by expanding a search radius, calculates a suitability of re-determined candidate hospitals, and re-determines an optimal transfer hospital on the basis of the calculated suitability of each candidate hospital.

9. The optimal transfer hospital determining server of claim 8, wherein when the re-determined suitability of the optimal transfer hospital is lower than a predetermined value, the following steps (1), (2), and (3) are continuously repeated until the re-determined suitability of the optimal transfer hospital becomes equal to or higher than the predetermined value:

(1) re-determining candidate hospitals by expanding a search radius;
(2) calculating a suitability of the re-determined candidate hospitals; and
(3) re-determining an optimal transfer hospital on the basis of the calculated suitability of each candidate hospital.

10. A method for determining a hospital to which each of a plurality of emergency patients is transported, the method comprising:

determining candidate hospitals;
generating weight information about each candidate hospital of each emergency patient;
determining an optimal transfer hospital of each emergency patient; and
inquiring the determined optimal transfer hospital about whether to accept the emergency patient, together with weight information.

11. The method of claim 10, wherein in the generating weight information, a weight for each candidate hospital of each emergency patient is calculated by transport distance based hospital modeling and patient information based modeling.

12. The method of claim 10, wherein when the determined optimal transfer hospital does not accept the emergency patient as a result of inquiring about whether to accept the emergency patient,
the weight information is re-generated after updating information about the emergency patient and candidate hospitals in real-time.

13. The method of claim 10, wherein after determining a final transfer hospital for each emergency patient, weight information about the determined final transfer hospital is generated.

14. The method of claim 10, wherein of the determining a final transfer hospital of each emergency patient includes:

acquiring status information about an emergency patient;
determining a severity of the patient on the basis of the acquired status information;
calculating emergency event possibility information on the basis of the acquired status information;
acquiring transport resources availability information about the determined candidate hospitals;
calculating the suitability of each candidate hospital on the basis of the determined severity of the patient, the acquired emergency event possibility information, and the transport resource availability information; and determining an optimal transfer hospital on the basis of the calculated suitability of each candidate hospital.

15. The method of claim 14, wherein the status information of the emergency patient includes at least one of biosignal information, age information, complained symptom information, existing medical history information, consciousness information, and electrocardiogram information,

the emergency event possibility information includes at least one of intensive care unit hospitalization possibility information, STEMI possibility information, UA + NSTEM possibility information, LVO possibility information, cerebral infarction and cerebral hemorrhage possibility information, return of spontaneous circulation possibility information, and cardiac arrest recurrence possibility information, and

the transport resource availability information includes at least one of real-time traffic information, location information of each candidate hospital, current position information, available sickbed information of each candidate hospital, duty doctor information of each candidate hospital, facility information of each candidate hospital, air ambulance location information, and air ambulance operation information.

16. The method of claim 15, further comprising:

when the determined suitability of the optimal transfer hospital is lower than a predetermined value,
re-determining candidate hospitals by expanding a search radius;
calculating a suitability of the re-determined candidate hospitals; and
re-determining an optimal transfer hospital on the basis of the calculated suitability of each candidate hospital.

17. The method of claim 16, wherein when the re-determined suitability of the optimal transfer hospital is lower than a predetermined value, the following steps (1), (2), and (3) are continuously repeated until the re-determined suitability of the optimal transfer hospital becomes equal to or higher than the predetermined value:

(1) re-determining candidate hospitals by expanding a search radius;
(2) calculating a suitability of the re-determined candidate hospitals; and
(3) re-determining an optimal transfer hospital on the basis of the calculated suitability of each candidate hospital.

18. The method of claim 14, further comprising:

determining whether to utilize an air ambulance to transport the emergency patient on the basis of at least one of the determined location information of the optimal transfer hospital, real-time traffic information, and air ambulance operation information, and

determining an optimal handover point on the basis of at least one of the determined location information of the optimal transfer hospital, real-time traffic information, and air ambulance operation information when the air ambulance is utilized to transport the emergency patient.

19. A server for determining a hospital to which each of a plurality of emergency patients is transported, comprising: a control unit which determines candidate hospitals, generates weight information about candidate hospitals of each emergency patient, determines an optimal transfer hospital of each emergency patient, and inquires the determined optimal transfer hospital about whether to accept the emergency patient together with the weight information.

20. The server of claim 19, wherein the control unit determines a severity of a patient on the basis of the acquired status information of the emergency patient, calculates emergency event possibility information, calculates a suitability of each candidate hospital on the basis of the determined severity of the patient, emergency event possibility information, and the transport resource availability information, and determines an optimal transfer hospital on the basis of the calculated suitability of each candidate hospital, and

the server further includes a storage unit which stores the status information of the emergency patient, the emergency event possibility information, and the transport resource availability information,

the status information of the emergency patient includes at least one of biosignal information, age information, complained symptom information, existing medical history information, consciousness information, and electrocardiogram information,

the emergency event possibility information includes at least one of intensive care unit hospitalization possibility information, STEMI possibility information, UA + NSTEM possibility information, LVO possibility information, cerebral infarction and cerebral hemorrhage possibility information, return of spontaneous circulation possibility information, and cardiac arrest recurrence possibility information, and

the transport resource availability information includes at least one of real-time traffic information, location information of each candidate hospital, current position information, available sickbed information of each candidate hospital, duty doctor information of each candidate hospital, facility information of each candidate hospital, air ambulance location information, and air ambulance operation information.

AMBULANCE
DEVICE
(1000)

EMERGENCY
MEDICAL
SERVER
(2000)

AMBULANCE

REAL-TIME RESOURCE
INFORMATION

EMERGENCY AI
SERVER
(3000)

EMERGENCY AI CLOUD
(LEARNING, SELECTING)

FIG. 1

DETERMINE CANDIDATE HOSPITALS (S210)

ACQUIRE STATUS INFORMATION ABOUT EMERGENCY PATIENT (S220)

DETERMINE SEVERITY OF PATIENT(S230)

CALCULATE EMERGENCY EVENT POSSIBILITY INFORMATION (S240)

ACQUIRE TRANSPORT RESOURCES AVAILABILITY INFORMATION (S250)

CALCULATE SUITABILITY OF EACH CANDIDATE HOSPITAL (S260)

DETERMINE FINAL TRANSFER HOSPITAL (S270)

FIG. 2

RE-DETERMINE CANDIDATE HOSPITALS
BY EXPANDING SEARCH RADIUS (S310)

CALCULATE SUITABILITY OF
RE-DETERMINED CANDIDATE HOSPITAL (S320)

RE-DETERMINE FINAL TRANSFER HOSPITAL (S330)

## FIG. 3

GENERATE WEIGHT INFORMATION OF EMERGENCY PATIENTS (S410)

DETERMINE FINAL TRANSFER HOSPITAL
OF EACH EMERGENCY PATIENT (S420)

PROPOSE OPTIMAL TRANSFER HOSPITAL
TOGETHER WITH WEIGHT INFORMATION (S430)

DETERMINE WHETHER TO ACCEPT PATIENT (S440)

UPDATE EMERGENCY PATIENT
AND HOSPITAL INFORMATION IN REAL-TIME (S450)

## FIG. 4

DETERMINE WHETHER TO UTILIZE AIR AMBULANCE (S510)

DETERMINE OPTIMAL HANDOVER POINT (S520)

## FIG. 5

## FIG. 6

EMERGENCY ARTIFICIAL
INTELLIGENCE (AI) SERVER
(3000)

CONTROL
UNIT
(3000)

NETWORK
CONNECTION UNIT
(3100)

STORAGE UNIT
(3200)

FIG. 7

# EP 4 027 352 A1

<table>
<tr><td colspan="2" align="center"><b>INTERNATIONAL SEARCH REPORT</b></td><td colspan="2">International application No.<br><br><b>PCT/KR2020/011872</b></td></tr>
</table>

**A.   CLASSIFICATION OF SUBJECT MATTER**

**G16H 40/20**(2018.01)i; **G16H 80/00**(2018.01)i; **G06Q 50/22**(2012.01)i; **G16H 50/50**(2018.01)i; **G16H 50/70**(2018.01)i; **G06Q 10/06**(2012.01)i

According to International Patent Classification (IPC) or to both national classification and IPC

**B.   FIELDS SEARCHED**

Minimum documentation searched (classification system followed by classification symbols)

G16H 40/20; G06F 19/00; G06Q 50/00; G06Q 50/22; H04L 29/08; G16H 80/00; G16H 50/50; G16H 50/70; G06Q 10/06

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 응급(emergency), 환자(patient), 이송(transfer), 병원(hospital) 및 최적(optimum)

**C.   DOCUMENTS CONSIDERED TO BE RELEVANT**

| Category* | Citation of document, with indication, where appropriate, of the relevant passages | Relevant to claim No. |
|---|---|---|
| Y | JP 5375864 B2 (OKI ELECTRIC IND. CO., LTD.) 25 December 2013. See paragraphs [0016], [0023], [0046], [0051] and [0113]. | 1,2,5-7 |
| A | | 3,4,8-20 |
| Y | KR 10-2007-0043337 A (INDUSTRY-ACADEMIC COOPERATION FOUNDATION, YONSEI UNIVERSITY) 25 April 2007. See paragraph [0110]. | 1,2,5-7 |
| A | KR 10-2013-0083067 A (KYUNGPOOK NATIONAL UNIVERSITY INDUSTRY-ACADEMIC COOPERATION FOUNDATION) 22 July 2013. See paragraphs [0005]-[0013] and figure 1. | 1-20 |
| A | KR 10-2011-0127537 A (THE CATHOLIC UNIVERSITY OF KOREA INDUSTRY-ACADEMIC COOPERATION FOUNDATION) 25 November 2011. See paragraphs [0005]-[0018]. | 1-20 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **16 December 2020** | **17 December 2020** |

| Name and mailing address of the ISA/KR | Authorized officer |
|---|---|
| **Korean Intellectual Property Office**<br>**Government Complex Daejeon Building 4, 189, Cheongsa-ro, Seo-gu, Daejeon, Republic of Korea**<br>**35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2020/011872** |

**C.**      **DOCUMENTS CONSIDERED TO BE RELEVANT**

| Category* | Citation of document, with indication, where appropriate, of the relevant passages | Relevant to claim No. |
| --- | --- | --- |
| A | US 2018-0137246 A1 (HEFEI UNIVERSITY OF TECHNOLOGY) 17 May 2018. See abstract and figure 1. | 1-20 |

Form PCT/ISA/210 (second sheet) (July 2019)

**INTERNATIONAL SEARCH REPORT**
**Information on patent family members**

International application No.

**PCT/KR2020/011872**

| Patent document cited in search report | | | Publication date (day/month/year) | Patent family member(s) | | | Publication date (day/month/year) |
|---|---|---|---|---|---|---|---|
| JP | 5375864 | B2 | 25 December 2013 | JP | 2012-208667 | A | 25 October 2012 |
| KR | 10-2007-0043337 | A | 25 April 2007 | KR | 10-0759916 | B1 | 18 September 2007 |
| KR | 10-2013-0083067 | A | 22 July 2013 | None | | | |
| KR | 10-2011-0127537 | A | 25 November 2011 | KR | 10-1222379 | B1 | 15 January 2013 |
| US | 2018-0137246 | A1 | 17 May 2018 | CN | 106506502 | A | 15 March 2017 |
| | | | | CN | 106506502 | B | 22 September 2017 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100800026